# DEMANDE DE BREVET EUROPEEN

(11) **EP 1 829 520 A1**
(43) Date de publication de la demande: **05.09.2007**
(21) Numéro de dépôt: 07300843.5
(22) Date de dépôt: 01.03.2007
(51) Int. Cl.: A61K 6/083, A61K 6/087

(54) **Elément prothétique dentaire antimicrobien et procédé de fabrication**

(30) Priorité: 02.03.2006 FR 0650740
(71) Demandeur: Société de Recherches Techniques Dentaires - R.T.D., 38120 Saint Egrève (FR)
(72) Inventeur: Reynaud, Pierre-Luc, 38410, VAULNAVEYS LE HAUT (FR); Chu, Manh-Quynh, 38120, FONTANIL CORNILLON (FR); Piet, Gérard, 38830, SAINT PIERRE D'ALLEVARD (FR); Bedel, Laurent, 38950, QUAIX EN CHARTREUSE (FR)
(74) Mandataire: Denjean, Eric

(57) **Abrégé**

Elément prothétique dentaire obtenu par usinage d'un profilé en matériau composite comprenant des fibres noyées dans une matrice contenant de la résine, caractérisé en ce qu'il est recouvert sur tout ou partie de sa surface, d'au moins une couche contenant au moins un agent ayant un effet antimicrobien.

## Description

L'invention a pour objet un élément prothétique dentaire réalisé en matériau composite, ledit élément présentant des propriétés antimicrobiennes. L'invention concerne également le procédé de fabrication dudit élément prothétique dentaire.

Par "élément prothétique dentaire", on désigne notamment, mais de façon non limitative, les tenons, les renforts de bridge, à l'exception des implants qui sont des éléments destinés à être insérés directement dans l'os maxillaire.

Dans la suite de la description, l'invention est plus particulièrement décrite en relation avec un tenon dentaire en matériau composite.

Les tenons dentaires sont utilisés pour la reconstitution de dents dépulpées. On distingue deux types de tenons, respectivement :
- les tenons métalliques ou céramiques, et
- les tenons composites.

Les tenons métalliques sont en général réalisés en acier inoxydable. Ils ont pour principaux inconvénients d'être sujets à des phénomènes de corrosion. En outre, ils présentent un module d'élasticité transversale différent de celui de la dentine pour conduire, à terme, à une désolidarisation du tenon.

Pour résoudre ces problèmes, on a proposé des tenons fabriqués en matériau composite tels que ceux décrits notamment dans le document EP-A-432 001 du Demandeur. Ces tenons sont en pratique constitués de fibres longues unidirectionnelles de verre ou de carbone noyées dans une matrice de résine thermodurcissable. De manière générale, la proportion de fibres longues, quelque soit la matière choisie, représente de 55 à 70% en volume du tenon, le complément à 100% étant occupé par la matrice.

Pour assurer le maintien du tenon dans la racine, celui-ci doit être collé, puis scellé dans ladite racine. Le praticien comble ensuite les espaces restants au moyen d'une pâte composite. La prothèse ou couronne vient ensuite recouvrir l'ensemble et est maintenue en position par ajout supplémentaire d'un ciment, éventuellement associé à un adhésif.

Malgré le soin apporté aux différentes étapes cliniques lors de la reconstitution de la dent, on observe parfois des récidives de caries conduisant au mieux, à un descellement de la prothèse nécessitant un retraitement complet de la racine, au pire, à une extraction de la dent.

Ces récidives de caries sont dues à une infiltration de germes pathogènes, tels que *Streptococcus mutans, Enterococcus faecalis,* voire dans certains cas, *Staphylococcus aureus.* Une telle infiltration peut être due à des pertes d'étanchéité marginale entre la prothèse, la dentine et le matériau de comblement. Des foyers de bactéries peuvent également subsister lorsque le mordançage de la dentine est mal exécuté et laisse apparaître des débris. Les germes peuvent enfin profiter d'obstacles naturels comme des contre-dépouilles, faisant office de réceptacle.

Pour résoudre ce problème, plusieurs solutions ont été développées telles que des adhésifs ou des ciments antimicrobiens, mais également des solutions de mordançage.

Ainsi, le document US-A-5 385 728 décrit une composition acide de mordançage contenant en tant qu'agent antimicrobien, du chlorure de benzalkonium.

Le document US-A-5 968 253 a pour objet un ciment dentaire à base de phosphate de calcium pouvant contenir un agent antimicrobien sous forme d'un antibiotique ou d'un antiamébique.

Le document US-A-6 326 417 concerne une composition dentaire antimicrobienne utilisée comme adhésif, ciment ou pâte composite de reconstitution contenant des composés d'acide salicylique ou de sulfamide, éventuellement sous forme dérivés.

Les documents US-A-5 408 022, US-A-5 494 987, US-A-5 733 949 décrivent des compositions à base de polymères possédant des propriétés antibactériennes après polymérisation, utilisées pour la mise en oeuvre de peau artificielle, cathéter, lentille de contact, mais également produit dentaire, tel que adhésif, résine, composite. L'agent antimicrobien est un monomère polymérisable, dont le groupement actif est à base de sel quaternaire (chlore, brome).

Le document US-A-6 355 704 décrit un adhésif dentaire antimicrobien bi-composants. Un des composants correspond à un monomère polymérisable antimicrobien présentant un groupe cationique sélectionné dans les bases d'ammonium, pyridinium et phosphonium.

Le document US-A-6 924 325 décrit une composition dentaire antimicrobienne pouvant être utilisée comme composition de restauration endodontique, orthodontique ou prothétique. Cette composition contient comme principe actif, une poudre de verre céramique / zinc-argent ou une poudre zéolite à base d'argent.

D'autres types de produits que ceux précités précédemment présentant des propriétés antimicrobiennes ont été développés. Il s'agit notamment de compositions utilisées pour le blanchiment des dents, telles que décrites dans les documents US-A-5 985 249, US-A-6 036 943, US-A-6 309 625, US-A-6 368 576 dans lesquels l'agent antimicrobien est sélectionné parmi la chlorhexidine, les dérivés de benzoate, la tétracycline ou les composés fluorés.

Le document US-A-6 365 130 décrit un chewing-gum contenant des particules céramiques antimicrobiennes à base de cations métalliques.

Le document US-A-6 267 590 se rapporte à un bracket et un fil orthodontique traités par dépôt d'un polymère céramique zéolite d'argent.

Le document US-A-6 113 993 se rapporte à une méthode de traitement d'un implant dentaire, le traitement consistant à appliquer à la surface de l'implant, un agent liant du type phosphate de calcium destiné à favoriser la croissance osseuse autour de l'implant. Une fois le traitement effectué, il est proposé de tremper les implants dans une solution d'un agent antimicrobien et ce, avant implantation. Après l'implantation le relargage du phosphate de calcium aura lieu en même temps que l'agent pour favoriser la croissance et/ou prévenir l'infection.

Dans un autre domaine, le document XP002295856 décrit des dispositifs médicaux du type cathéter recouverts d'argent sous la forme d'ions métalliques. Cette étude montre une efficacité antimicrobienne inégale en fonction des dispositifs traités.

A la connaissance du Demandeur, aucune solution n'a été proposée pour rendre un élément prothétique composite du type tenon notamment, antimicrobien pour lequel il existe un certain nombre de contraintes et en particulier :
- préservation de la flore buccale,
- maintien des caractéristiques mécaniques,
- niveau de transparence suffisant permettant la transmission des rayons lumineux provenant de la lampe à photopolymériser.

Le Demandeur a mis au point un élément prothétique dont tout ou partie de la surface est recouverte d'au moins une couche contenant un agent antimicrobien.

En d'autres termes, l'invention a pour objet un élément prothétique dentaire obtenu par usinage d'un profilé en matériau composite comprenant des fibres noyées dans une matrice contenant notamment de la résine.

Cet élément prothétique se caractérise en ce qu'il est recouvert, sur tout ou partie de sa surface, d'au moins une couche contenant au moins un agent ayant un effet antimicrobien.

Dans un mode de réalisation particulier, l'élément prothétique est un tenon. En pratique, le tenon comprend des fibres de renfort noyées dans une matrice comprenant de la résine. Il peut s'agir de fibres de verre, de carbone, de céramique ou encore de quartz.

En pratique, l'agent antimicrobien est un métal. Avantageusement, l'agent antimicrobien est choisi dans le groupe comprenant l'argent et le cuivre.

Pour améliorer l'adhésion et donc interdire le relargage de l'agent anti-microbien du tenon ou plus largement de l'élément prothétique, la couche contient également au moins une substance inorganique telle que par exemple un oxyde métallique. Dans ce cas de figure, le métal est appliqué sur la surface de l'élément prothétique, éventuellement en mélange avec l'oxyde métallique. Avantageusement, l'oxyde métallique est choisi dans le groupe comprenant l'oxyde d'aluminium, l'oxyde de baryum, l'oxyde de strontium, l'oxyde de zirconium.

En pratique, la couche comprenant l'agent anti-microbien a une épaisseur comprise entre 0,01 µm et 5µm, avantageusement 0,25 µm, cette épaisseur étant déterminée au niveau du contour le plus régulier du tenon. Pour une épaisseur inférieure, les propriétés antimicrobiennes sont moins bonnes. Au-delà, l'épaisseur devient trop importante pour permettre l'insertion satisfaisante du tenon ou engendre des problèmes inesthétiques.

Par ailleurs et selon une autre caractéristique, l'agent antimicrobien représente entre 0.001% à 1% en poids, avantageusement 0.25% en poids de la couche. Rapporté à l'élément prothétique et notamment au tenon, l'agent antimicrobien représente entre 0.001% et 5% en poids, avantageusement 0.05%.En dehors de cette fourchette, l'effet antimicrobien est insuffisant ou pas meilleur.

Dans un mode de réalisation avantageux, l'élément prothétique est non seulement anti microbien mais également radio opaque.

Pour rendre le produit visible aux rayons X, l'élément prothétique contient une substance radio-opaque.

L'homme du métier connaît plusieurs manières de rendre le produit radio-opaque.

Dans une première forme de réalisation, au moins un produit radio-opaque est incorporé dans la fibre. Dans ce cas, on peut utiliser des fibres à base par exemple d'oxyde de calcium ou encore d'oxyde de zirconium.

Dans une seconde forme de réalisation, au moins une substance radio-opaque est incorporée dans la matrice.

Une troisième possibilité, qui peut éventuellement être combinée à l'une ou l'autre, voire les deux solutions sus-mentionnées, est d'incorporer l'agent radio opaque dans la couche de surface. Avantageusement, on utilisera un même oxyde métallique permettant à la fois de conférer la radio opacité à l'élément prothétique et d'améliorer l'adhésion de l'agent anti-microbien.

En pratique, la substance radio-opaque est choisie dans le groupe comprenant les oxydes métalliques, tels que oxyde d'aluminium, oxyde de baryum, oxyde de strontium, oxyde de zirconium, les composés fluorés, tels que l'ytterbium et l'yttrium, seuls ou en mélange.

L'oxyde de zirconium est préféré lorsqu'il s'agit d'obtenir les deux propriétés, respectivement d'accrochage de l'agent anti microbien et de radio opacité.

Pour favoriser le collage, puis le scellement du tenon dans les racines par le biais d'un ciment composite, il est préférable de traiter le tenon au moyen d'un agent liant, en particulier du silane ou par un adhésif. En pratique, le praticien applique le silane extemporanément à la surface du tenon, c'est-à-dire au moment de sa mise en place dans la racine. Or, on sait que la silanisation d'un matériau composite est généralement médiocre du fait de l'absence d'accrochage efficace du silane

Or, le Demandeur a constaté que de manière tout à fait surprenante, l'adjonction de la substance inorganique interdisant le relargage de l'agent anti microbien dans la couche contenant ledit agent antimicrobien permettait en outre d'améliorer l'accroche du silane au tenon en vue de son collage. Les tenons peuvent alors être fournis au praticien sous forme silanisée, ce qui permet d'éviter au praticien d'avoir à effectuer une préparation extemporanée. En d'autres termes et dans un mode de réalisation avantageux, la couche contenant l'agent anti microbien contient également un agent liant tel que par exemple un silane.

En pratique, le silane est choisi dans le groupe comprenant le 3-(triméthoxysilyl)propyl méthacrylate, vinyltriméthoxysilane, 3-(glycidyloxy)propyl triméthoxysilane et 3-(triméthoxysilyl)propyl méthacrylate.

L'invention a également pour objet un procédé de fabrication de l'élément prothétique, en particulier du tenon précédemment décrit.

Selon ce procédé, on prépare tout d'abord un profilé en matériau composite comprenant des fibres noyées dans une matrice comprenant notamment de la résine.

Lorsque l'élément prothétique est un tenon, le profilé contient des fibres de renfort noyées dans une matrice de résine, par exemple une résine époxy.

En pratique, les fibres sont des fibres de verre, éventuellement radio-opaques, des fibres céramiques, ou encore des fibres de quartz.

Une fois le profilé obtenu, celui-ci est usiné pour obtenir la forme souhaitée de l'élément prothétique.

On applique alors sur la surface de l'élément prothétique, au moins une couche contenant au moins un agent ayant un effet antimicrobien.

Dans un mode de réalisation particulier, la couche contient en outre une substance inorganique, telle que par exemple un oxyde métallique. Cette substance inorganique remplit une première fonction qui est celle d'améliorer l'adhésion du métal à la surface de l'élément prothétique. Lorsque la substance inorganique est un oxyde métallique potentiellement radio-opaque, alors elle remplit une seconde fonction qui est celle de rendre l'élément prothétique, radio-opaque. Les différents composants d'une même couche peuvent être appliqués simultanément ou successivement.

Dans une étape additionnelle, on silanise l'élément prothétique c'est-à-dire qu'on applique sur tout ou partie de la couche contenant un agent antimicrobien au moins un agent liant tel qu'un silane.

La couche contenant l'agent antimicrobien et éventuellement une substance inorganique, est appliquée par la technique dite PVD (dépôt en phase vapeur) avantageusement par pulvérisation cathodique magnétron ou par technique CVD (Chemical Vapor Deposition).

En revanche, l'agent liant est en pratique appliquée par simple trempage, notamment dans une solution de silane.

L'invention et les avantages qui en découlent ressortiront mieux des exemples non limitatifs de réalisation suivants.

### Exemple 1

Les tenons sont obtenus après usinage d'un profilé contenant des fibres de verre E représentant un volume de 60% noyées dans une matrice de résine époxyde à base de Bisphénol A, la matrice représentant 40% en volume et ne contenant pas de charges radio-opaques.

### Dépôt d'une couche contenant de l'argent et du zircone sur la surface des tenons dentaires

Les dépôts sont réalisés simultanément par pulvérisation cathodique magnétron à partir d'une cible de zirconium et d'une cible d'argent. Les tenons fixés sur un support métallique sont positionnés à 10 cm des cibles. Avant dépôt, un vide de 4.10⁻³ Pa est réalisé dans le réacteur. De l'argon et de l'oxygène sont introduits pendant l'étape de dépôt. Les cibles sont pulvérisées par une décharge DC pulsé avec respectivement une puissance de 700 W et 300W pour la cible de zirconium et la cible d'argent sous une pression de 1 Pa.

La couche contenant de l'argent recouvre toute la surface des tenons, excepté une longueur de 2 à 3 mm que le praticien découpera lors de la mise en place.

L'épaisseur de dépôt obtenue est de l'ordre de 0.250µm (faible concentration d'argent) et elle n'a aucune influence sur les caractéristiques dimensionnelles du tenon. L'espace prévu pour le collage et le scellement est habituellement d'au moins 20µm au rayon.

Lors de ce dépôt, des échantillons diamètre 8mm du même matériau que celui employé pour usiner les tenons ont été également traités.

### Activité anti-microbienne:

Trois échantillons de diamètre 8mm revêtus sont soumis aux essais d'activité antimicrobienne en même temps que trois échantillons non traités utilisés comme témoin.

La méthode d'essais est inspirée de la norme Japanese Industrial Standard JIS Z 2801 « Antimicrobial products-Test for antimicrobial activity and efficacy ».

La souche bactérienne employée est le *Staphylococcus aureus.*

Tous les échantillons sont mis en incubation pendant 24h à 37°C. Les populations de *Staphylococcus aureus* sont ensuite décomptées. L'activité antimicrobienne de l'échantillon est égale à la différence de la moyenne des trois échantillons témoins moins la moyenne des trois échantillons à tester. L'activité antimicrobienne est égale au log de la différence entre le nombre de bactéries dénombrées sur l'échantillon témoin moins le nombre de bactéries dénombrées sur l'échantillon à tester. Les échantillons issus de cet exemple ont phagocyté totalement toutes les bactéries puisque l'on dénombre 0 germes après 24h d'incubation.

L'activité bactéricide est de:
- 5,28 log sur *Staphylococcus aureus,*
- 4.42 log sur *Streptococcus mutans,*
- 5.02 log sur *Enterococcus faecalis*

### Propriété mécanique :

Les tenons sont encastrés dans un support en bronze et inclinés à 45°. Une force est appliquée à une vitesse constante de 1mm/minute par une machine traction-compression. La force maximale à la rupture est ensuite enregistrée.

### Les résultats :

Tenons témoins non traités : 94 N ± 4N force maximale
Tenons traités : 95 N ± 6N force maximale

Les tenons traités dans cet exemple ont permis la polymérisation de l'adhésif comme les tenons témoins.

### Collage

Les tenons traités antimicrobiens sont ensuite trempés dans une solution contenant du silane MEMO pendant 15 minutes. Ils sont ensuite séchés dans une étuve pour évaporer le solvant et l'eau.

### Solution MEMO :

Solution éthanol contenant environ 10% d'eau acidifiée acide acétique
MEMO : 3-(Triméthoxysilyl)propyl méthacrylate 5% en poids

Les tenons sont ensuite testés par la méthode dite « push-out » du Demandeur. La valeur d'adhésion en cisaillement est exprimée en MPa.

Les résultats de collage obtenus à partir des tenons fabriqués selon cet exemple ont été améliorés : 32 MPa au lieu de 26.9 MPa avec un adhésif et 32 MPa au lieu de 23 MPa sans adhésif. Les tenons non traités antimicrobiens et silanisés ont atteint seulement une valeur de 29 MPa.

Le protocole de collage ou scellement a été réduit d'une étape.

En effet il n'est plus nécessaire d'appliquer une couche d'adhésif sur le tenon puis de le photo-polymériser.

### Exemple 2

Les tenons identiques à l'exemple précédent sont traités dans les mêmes conditions sauf la durée d'exposition qui a été multipliée par trois.

L'épaisseur de dépôt obtenue est de l'ordre de 0.25µm proche de l'essai précédent mais la teneur en argent a été multipliée par dix.

Les échantillons traités sont soumis à l'activité antimicrobienne décrite précédemment.
Leur activité bactéricide est de 5,19 log sur *Staphylococcus aureus.*

Les résultats des essais de fracture à 45° et de transmission de lumière sont proches des résultats précédents et de ceux des tenons témoins.

En ce qui concerne les résultats des essais de collage, la valeur obtenue est 29.7 MPa au lieu de 32 MPa comme dans l'exemple précédent.

Ils confirment que les dépôts antimicrobiens permettent au moins d'égaler celle des tenons non traités et surtout que cette couche permet d'améliorer l'adhésion comparativement à celle d'un tenon collé avec un adhésif classique (26.9 MPa) et sans adhésif (23 MPa).

Les tenons traités par ce procédé permettent non seulement de posséder une activité antimicrobienne suffisante, sans présenter une toxicité secondaire non souhaitée tout en permettant de préserver une propriété adhésive forte voire améliorée en réduisant le protocole clinique d'une étape.

### Exemple 3

Les tenons identiques à ceux l'exemple 2 sont traités dans les mêmes conditions (temps de pulvérisation multiplié par dix) avec du cuivre.

L'épaisseur de dépôt obtenue est de l'ordre de 0.25µm proche de l'essai précédent.

Les échantillons traités sont soumis à l'activité antimicrobienne décrite précédemment.

Leur activité bactéricide est de 2,84 log sur *Streptococcus mutans.*

Les résultats des essais de fracture à 45° et de transmission de lumière sont proches des résultats précédents et de ceux des tenons témoins.

En ce qui concerne les résultats des essais de collage, la valeur obtenue est 34.9 MPa.

Ils confirment que les dépôts antimicrobiens permettent au moins d'égaler celle des tenons non traités et surtout que cette couche permet d'améliorer l'adhésion comparativement à celle d'un tenon avec un adhésif classique (26.9 MPa) et sans adhésif (23 MPa).

La présente invention présente un grand nombre d'avantages parmi lesquels :
■ action antimicrobienne localisée sans relargage d'éléments pouvant détruire la flore naturelle buccale ou disséminer des éléments secondaires dans tout l'organisme,
■ préservation des propriétés mécaniques (la technique de dépôt, son épaisseur ainsi que sa nature n'ont pas d'influence négative sur le tenon),
■ préservation de la transparence du tenon en matériau composite fibré permettant la transmission des rayons lumineux provenant de la lampe à photopolymériser,
■ respect de la rugosité de surface initiale du tenon,
■ compatible avec une silanisation,
■ conservation voire amélioration des valeurs d'adhésion entre le tenon et le ciment sans faire appel à un adhésif ou primer.

## Revendications

1. Elément prothétique dentaire obtenu par usinage d'un profilé en matériau composite comprenant des fibres noyées dans une matrice contenant de la résine, **caractérisé en ce qu'**il est recouvert sur tout ou partie de sa surface, d'au moins une couche contenant au moins un agent ayant un effet antimicrobien.

2. Elément prothétique selon la revendication 1, **caractérisé en ce que** l'agent antimicrobien est un métal.

3. Elément prothétique selon la revendication 1, **caractérisé en ce que** le métal est l'argent ou le cuivre.

4. Elément prothétique selon l'une des revendications précédentes, **caractérisé en ce que** la couche contient également une substance inorganique.

5. Elément prothétique selon la revendication 4, **caractérisé en ce que** la substance inorganique est un oxyde métallique.

6. Elément prothétique selon l'une des revendications précédentes, **caractérisé en ce que** la couche contenant l'agent anti-microbien a une épaisseur comprise entre 0,01 µm et 5µm.

7. Elément prothétique selon l'une des revendications précédentes, **caractérisé en ce que** la couche contenant un agent antimicrobien contient en outre au moins une substance radio-opaque.

8. Elément prothétique selon la revendication 7, **caractérisé en ce que** la substance radio-opaque est choisie dans le groupe comprenant les oxydes métalliques, tels que oxyde d'aluminium, oxyde de baryum, oxyde de strontium , oxyde de zinc, oxyde de zirconium, les composés fluorés, tels que l'ytterbium et l'yttrium.

9. Elément prothétique selon l'une des revendications précédentes, **caractérisé en ce que** la couche contient en outre un silane.

10. Procédé pour la fabrication d'un élément prothétique dentaire composite selon lequel :
- on prépare un profilé contenant des fibres noyées dans une matrice comprenant de la résine,
- on usine le profilé à la forme souhaitée de l'élément prothétique,
- on applique sur l'élément prothétique obtenu au moins une couche contenant au moins un agent ayant un effet antimicrobien.

11. Procédé selon la revendication 10, **caractérisé en ce que** la couche contenant au moins un agent ayant un effet antimicrobien contient en outre une substance inorganique.

12. Procédé selon l'une des revendications 10 à 11, **caractérisé en ce que** la couche contenant au moins un agent ayant un effet antimicrobien contient en outre une substance radio-opaque.

13. Procédé selon la revendication 12, **caractérisé en ce que** la substance radio-opaque est choisie dans le groupe comprenant les oxydes métalliques, tels que oxyde d'aluminium, oxyde de baryum, oxyde de strontium, , oxyde de zinc, oxyde de zirconium, les composés fluorés, tels que l'ytterbium et l'yttrium.

14. Procédé selon l'une des revendications 10 à 13, **caractérisé en ce que** la couche contenant au moins un agent ayant un effet antimicrobien est appliquée par la technique dite PVD (dépôt en phase vapeur), par pulvérisation cathodique magnétron ou par technique CVD (Chemical Vapor Deposition).

15. Procédé selon l'une des revendications 10 à 14, **caractérisé en ce que**, une fois la couche contenant un agent antimicrobien appliquée, on silanise l'élément prothétique.

16. Elément prothétique selon l'une des revendications 1 à 9, **caractérisé en ce qu'**il s'agit d'un tenon.
